# EUROPEAN PATENT SPECIFICATION

(11) **EP 0 967 214 B1**
(45) Date of publication and mention of the grant of the patent: **23.06.2004**
(21) Application number: 99304425.4
(22) Date of filing: 08.06.1999
(51) Int. Cl.: C07D 487/04, A61K 31/505

(54) **Intranasal formulations for treating sexual disorders**
Intranasale Zubereitungen zur Behandlung sexueller Störungen
Formulation intranasale pour le traitement des troubles sexuels

(30) Priority: 22.06.1998 GB 9813452; 24.09.1998 GB 9820837; 13.02.1999 GB 9903177
(43) Date of publication of application: 29.12.1999
(73) Proprietor: Pfizer Limited, Sandwich, Kent CT13 9NJ (GB); Pfizer Ireland Pharmaceuticals, Dun Laoghaire, County Dublin (IE)
(72) Inventor: Billotte, Anne, Sandwich, Kent, CT13 9NJ (GB); Dunn, Peter James, Sandwich, Kent, CT13 9NJ (GB); Henry, Brian Thomas, Sandwich, Kent, CT13 9NJ (GB); Marshall, Peter Vallance, Sandwich, Kent, CT13 9NJ (GB); Woods, Joanna Jayne, Sandwich, Kent, CT13 9NJ (GB)
(74) Representative: Moore, James William

(56) References cited:
- WO-A-94/28902
- WO-A-95/05172
- WO-A-99/27905
- DATABASE WPI Section Ch, Week 199905 Derwent Publications Ltd., London, GB; Class B02, AN 1999-059770 XP002119443 & WO 98 53819 A (MOCHIDA PHARM CO LTD), 3 December 1998 (1998-12-03)

## Description

This invention relates to intranasal formulations of cyclic guanosine 3', 5'-monophosphate phosphodiesterase type five (cGMP PDE5) inhibitors, including in particular the compound sildenafil, for the treatment of sexual disorders such as impotence. The invention also includes sildenafil mesylate and intranasal formulations thereof and its use in treating sexual disorders.

According to the specification of our International patent application W094/28902 we have discovered that compounds which are inhibitors of the cGMP PDE5 enzyme are potent and effective compounds for the treatment of male erectile dysfunction (MED, impotence) and for female sexual disorders. This discovery led to the development of the compound sildenafil (5-[2-ethoxy-5-(4-methylpiperazin-1-ylsulphonyl)phenyl]-1,6-dihydro-1-methyl- 3-propylpyrazolo[4,3-d]pyrimidin-7-one) (VIAGRA™) which has proved to be outstandingly successful as the first orally effective treatment for MED. W098/53819 which was published on 3 December 1998 (after the priority date of the present invention) claims intranasal compositions of cGMP phosphodiesterase inhibitors, including sildenafil, for treating erectile dysfunction.

The intranasal route has previously been employed as a mode of administration for certain pharmaceutical products. The absorption rate of an agent from the nasal cavity is dependent on a number of variables; however two key factors are the surface area available for absorption and the local blood flow of the nasal cavity. The available surface area for absorption is dictated by the nasal cavity airflow resistance which is under the control of a dense capillary bed of erectile carvemous tissue in the nasal cavity. Vasodilation of these tissues leads to nasal congestion or rhinitis, for example, which increases resistance to air flow and reduces the available surface area for drug absorption. However, vasodilation can also increase bloodflow and enhance absorption by increasing the rate of remove of the drug from the site of absorption.

Vasodilation has been shown to have a wide range of effects on nasal drug absorption. Increased nasal blood flow, nasal inflammation and rhinitis have been shown to have no effect on the intranasal absorption of some agents, however these effects have also been shown to both increase and decrease the absorption of other agents. Thus, it is unclear whether vasodilation will lead to enhanced or reduced nasal absorption following intranasal dosing of a drug.

Inhibitors of the PDE5 enzyme are potent vasodilators. PDE5 has been shown to be located in the capillary bed of the nasal cavity. Inhibitors of this enzyme might therefore be expected to lead to local vasodilation and nasal congestion. Intranasal administration of a PDE5 inhibitor would be anticipated to increase local vasodilation and could cause nasal congestion. Local increased blood flow may enhance the absorption rate of the drug but vasolidation could cause nasal congestion which may decrease the available surface area for absorption. Moreover the drug could cause local irritation. Thus the effectiveness and acceptability of this route of administration for these agents is difficult to predict.

We have surprisingly discovered that sildenafil can be successfully administered by the intranasal route and moreover the drug is surprisingly more rapidly absorbed following intranasal administration compared to the corresponding oral dose, leading to a more rapid onset of action and efficacy at lower doses. Although, as explained above PDE5 inhibitors have the potential to cause nasal congestion, this effect was not sufficient to inhibit the rapid absorption of the drug.

A further factor influencing the ability of a product to be absorbed following nasal administration is aqueous solubility. This enables the compound to dissolve in the mucosal tissue lining the nasal cavity when administered as a powder. Moreover, since only a small volume of a nasal formulation (such as an aqueous spray) can be applied, for administration as a solution, it is important to be able to achieve a sufficiently high concentration of the active ingredient to ensure that sufficient drug can be delivered to each nostril.

According to the present invention, we have discovered that one particular salt of sildenafil, sildenafil mesylate, has unexpectedly high aqueous solubility and this makes it particularly suitable for use in aqueous intranasal formulations. Sildenafil mesylate is a novel salt form of sildenafil and forms the primary aspect of this invention. We have also discovered that sildenafil mesylate forms a crystalline mono and dihydrate which have advantages in terms of their long term stability on storage and this forms a further feature of this aspect of the invention.

As well as being particularly suited to intranasal administration, sildenafil mesylate may be administered by a number of other routes where high aqueous solubility is an advantage.

Intranasal formulations are well known in the art and can either be powder formulations or more commonly nasal sprays. Such sprays typically comprise a solution of the active drug in physiological saline or other pharmaceutically suitable carrier liquids. Various nasal spray compression pumps are also well known in the art and can be calibrated to deliver a predetermined dose of the active drug.

The nasal formulations should deliver a dose of cGMP-PDE5 inhibitor of from 1 to 100mg, more preferably 5 to 20mgs per shot which can be given as one or more shots per nostril.

For solution formulations typical volumes used are 25 to 200µL, more preferably 75 to 150µL per dose in each nostril. The intranasal solution formulations can be administered as drops from a nasal dropper bottle or as aerosols after being applied from squeeze bottles, single unit dose or metered-dose pump sprays. To avoid nasal irritation the formulations are preferably buffered to pH3-8, more preferably 4 to 7 using standard buffer systems, such as citrate, lactate or phosphate buffers to control the pH. In addition osmolarity must be adjusted so that the formulation is isotonic using standard osmogens (e.g. sodium chloride, mannitol or glucose).

Additional stabilisers may be required to improve chemical stability of the formulations; i.e. anti-oxidants such as sodium metabisulfite, sodium bisulfite or tocopherol, or metal chelators such as ethylenedaminetetraacetic acid.

Single unit-dose spray can be prepared aseptically or terminally sterilised to produce a sterile final product. Alternatively, multi-dose metered valve pump systems can be maintained free of microbial contamination with the use of chemical preservatives (e.g. benzalkonium chloride or benzyl alcohol).

Flavouring, perfumes and humectants may also be added to improve the patient acceptability of the formulations.

One particular and preferred formulation comprises a solution of the active cGMP PDE5 inhibitor in 5% weight/volume aqueous glycerine.

In another particular and preferred aspect of the invention we have discovered that by using a solubility enhancer it is possible to further improve the aqueous solubility of sildenafil mesylate. Examples of suitable solubility enhancers include xanthines (e.g. caffeine), vitamins (e.g.nicotinamide) and pharmaceutical excipients (e.g. vanillin and benzyl alcohol). Combination of any of these agents is also possible.

Preferred as solubility enhancing agents are caffeine (preferably at a concentration of from 1.0 to 2.5% weight/vol); nicotinamide (preferably 3.0 to 20.0% weight/vol); vanillin (preferably 0.5 to 2.5% weight/vol); and benzyl alcohol (preferably 0.5 to 2.5% weight/vol). A combination of nicotinamide and vanillin is also preferred. By using such solubility enhancing agents, it is possible to increase the solubility of sildenafil mesylate in water from approximately 60 mg/ml to in excess of 100 mg/ml. This alllows a more concentrated solution to be administered facilitating a rapid onset of action and reducing irritancy. One particular and preferred formulation comprises sildenafil mesylate 100 mg/ml and caffeine 15 mg/ml in a buffered aqueous solution. The pH of the solution is preferably adjusted to pH 3-5, preferably to pH 4.2 by the addition of a base e.g. sodium hydroxide.

The formulations are conveniently prepared by dissolving sildenafil mesylate, the solubility enhancer and buffer in water, adjusting the pH if necessary, sterilising by filtration or autoclave and aseptically filling into spray bottles or other dispensers. Alternatively sildenafil free base can be added to an aqueous solution of methane sulphonic acid and solubility enhancer (eg caffeine), stirred until dissolved, buffer added and the pH adjusted prior to sterilising and filling as before.

Powder formulations can overcome stability issues associated with liquid formulations and are not limited by solubility, thus higher doses can be delivered into the nasal cavity. Sildenafil mesylate can be formulated as powder formulation to be insufflated into the nose utilising specialised drug delivery devices (available from commercial manufacturers such as Mait Spa, Italy; Valois SA, France; Pfeiffer, Germany or Orion, Finland). The powder can be placed in hard gelatine capsules, foil blisters or as an integral part of the device for delivery of single unit doses. Alternatively, multi-dose dry powder systems are also available.

The particle size of the powder is an important factor for successful delivery to the nasal cavity. Powders with particle size <1µm tend to be carried through the nose and inhaled into the lungs, whereas larger particles may not have a sufficient dissolution rate to allow absorption during the short nasal residence time. Preferred particle size distribution for powder formulations in accordance with the present invention is 1 - 100µm, more preferably 5 - 40µm.

In addition, carrier powders such as lactose and dextrose are often blended with the drug powder to aid manufacture and dose reproductbility on intranasal administration.

Thus the invention also includes an intranasal pharmaceutical formulation for the treatment of male erectile dysfunction or female sexual disorders which comprises sildenafil mesylate together with a pharmacticually acceptable diluent or carrier in a form adapted for intranasal administration.

The effectiveness of the intranasal formulations of the present invention were evaluated in dogs. Four fasted dogs were lightly anaesthetised, each received 5mg of the cGMP-PDE5 inhibitor in both nostrils. The drug was administered both as a powder and as a solution. Plasma levels of the active agent were measured and compared with plasma levels obtained when the dogs were previously orally dosed with the agent.

Results from these studies showed that intranasal administration of sildenafil led to a rapid and significantly higher blood plasma levels than obtained following oral administration. Sildenafil mesylate was particularly effective.

Thus a solution dose of 0.7mg/kg of sildenafil mesylate administered by the intranasal route to four dogs gave a mean peak blood plasma levels of 407ng/ml after a period of 5 minutes. This can be compared with an oral dose of 1.4mg/kg of sildenafil citrate which gave mean peak blood plasma levels of 204ng/ml after 136 minutes.

These findings have been confirmed in man where studies in volunteers have shown that blood plasma levels of sildenafil comparable with oral dosing can be obtained following intranasal administration of sildenafil mesylate, with peak blood plasma levels occurring 5-15 minutes after administration.

The following examples, illustrate preparation of the formulations of the invention as well as the preparation of sildenafil mesylate and the crystalline hydrates thereof.

### EXAMPLE 1

### INTRANASAL SOLUTION FORMULATIONS

Intranasal solution formulations were prepared of the following composition:

| | | |
|---|---|---|
| 1. | Sildenafil mesylate | 50mg |
| | Water for injections to | 1 mL. |
| | | |
| 2. | Sildenafil mesylate | 50mg |
| | Glucose | 50mg |
| | Water for injection to | 1 mL |
| | | |
| 3. | Sildenafil mesylate | 50mg |
| | Glucose | 50mg |
| | Benzyl alcohol | 10mg |
| | Water for injections to | 1mL |
| | | |
| 4. | Sildenafil mesylate | 25mg |
| | 5% w/v aqueous glycerine to | 1mL |
| | | |
| 5. | Sildenafil mesylate | 50mg |
| | 5% w/v aqueous glycerine to | 1mL |

The solutions were aseptically filtered and filled into plastic nasal spray bottles.

### EXAMPLE 2

### INTRANASAL SOLUTION FORMULATION

A solution was prepared containing the following:

| | |
|---|---|
| Sildenafil mesylate | 10g |
| Caffeine | 1.5g |
| Sodium dihydrogen phosphate | 0.69g |
| Distilled water | to 100ml |

The solution was stirred to dissolve the ingredients and the pH adjusted to 4.2 by the addition of 1M sodium hydroxide solution. The solution was sterilised by ultrafiltration or by autoclave at 120°C for 20 minutes and the cooled solution was aseptically filled into monodose nasal spray devices to deliver a unit dose of 100 microlitres.

Compositions were similarly prepared using nicotinamide (5.0g); vanillin (1.5g) or benzyl alcohol (1.5g) instead of caffeine.

### EXAMPLE 3

### INTRANASAL POWDER FORMULATIONS

Intranasal powder formulation was prepared of the following composition:

| | |
|---|---|
| Sildenafil Mesylate | 5mg(A) |
| Lactose | 35mg |

The composition was milled to an average particle size of 20µm and filled into a gelatin capsule for use with a commercial nasal insufflator.

### EXAMPLE 4

### Preparation of 5-[2-ethoxy-5-(4-methylpiperazin-1-ylsulphonyl)phenyl]-1,6-dihydro-1-methyl- 3-propylpyrazolo[4,3-d]pyrimidin-7-one) methanesulphonate salt (sildenafil mesylate)

5-[2-Ethoxy-5-(4-methylpiperazin-1-ylsulphonyl)phenyl]-1,6-dihydro-1-methyl-3-propylpyrazolo [4,3-d]pyrimidin-7-one)' (100g, 0.21 mol) was dissolved in boiling acetone (3000 ml). Methanesulphonic acid (14.9 ml, 0.23 mol) was added to the hot acetone solution. Within 10 seconds a precipitate formed. The mixture was allowed to cool and granulate for 48 hours. The title product was collected by filtration and dried in vacuum to give a white crystalline solid (116.0g, 96.8%), m.p. 272-274°C.

Found: C, 48.33; H, 5.99; N, 14.68. C₂₃H₃₄N₆O₇S₂ requires C, 48.41; H, 6.00; N, 14.73 % δ (CD₃SOCO₃)² 0.92 (3H, t), 1.33 (3H, t), 1.73 (2H, heptet), 2.29 (3H,s), 2.77 (2H, t), 2.79 (3H, s), 3.16 (2H, br), 3.3-3.57 (4H, br), 3.8 (2H, br), 4.16 (3H, s), 4.20(2H, q), 7.4 (1H, d), 7.88 (1H, dd), 7.90 (1H, s), 9.44 (1H, br).
2. The nmr data was obtained on a Varian Unity 300 Spectrometer which was operating at 300 MHz.

### EXAMPLE 5

### Preparation of 5-[2-ethoxy-5-(4-methylpiperazin-1-ylsulphonyl)phenyl]-1,6-dihydro-1-methyl-3-propylpyrazolo [4,3-d]pyrimidin-7-one) methanesulphonate dihydrate (sildenafil mesylate dihydrate).

5-[2-Ethoxy-5-(4-methylpiperazin-1-ylsulphonyl)phenyl]-1,6-dihydro-1-methyl-3-propylpyrazolo[4,3-d]pyrimidin-7-one)¹ (100g, 0.21 mol) was dissolved in 2-butanone (1500 ml) at 55°C and heated to reflux. A solution of methanesulphonic acid (14.9 ml, 0.23 mol) in water (75 ml) was added to the hot 2-butanone solution. After 20 minutes a precipitate formed. The mixture was allowed cool and granulate at ambient temperature for 6 hours. The title product was collected by filtration and air dried to give a white crystalline solid (119.5g, 93.5%). This material dehydrates on heating in a melting point apparatus to give the anhydrous form which melts at 272-274°C. A small sample was carefully dried for nmr and Karl Fischer analysis.
1. Prepared as described in US patent 5,250,534 and European Patent 0463756

Found: δ (CD₃SOCD₃)² 0.93 (3H, t), 1.33 (3H, t), 1.73 (2H, sextet), 2.29 (3H, s), 2.62 (2H,br), 2.76 (2H, t), 2.79 (3H,s), 3.15 (2H, br), 3.29 (HDO peak), 3.45 (2H, br), 3.78 (2H, br), 4.15 (3H, s), 4.21 (2H,q), 7.4 (1H,d), 7.9 (1H, s), 7.8 (H, dd), 9.43 (1H, br), 12.21 (1H,s).
Water content (by Karl Fischer)³= 6.7 % (theoretical for dihydrate= 5.93%)
3. Karl Fischer data was obtained from a Metrohm 701 KF Titrino Instrument.

### EXAMPLE 6

### Preparation of 5-[2-ethoxy-5-(4-methylpiperazin-1-ylsulphonyl)phenyl]-1,6-dihydro-1 methyl-3-propylpyrazolo [4,3-d]pyrimidin-7-one) methanesulphonate monohydrate (sildenafil mesylate monohydrate).

Anhydrous 5-[2-ethoxy-5-(4-methylpiperazin-1-ylsulphonyl)phenyl]-1,6-dihydro-1-methyl-3-propylprazolo[4,3-d]pyrimidin-7-one) methanesulphonate (1.082g) was suspended in a solution of acetone (19.4 ml) and water (0.6 ml). The suspension was allowed to stir for 1 week at room temperature and a further 55 microlitres of water added, followed a few days later by 30 microlitres of water and then 15 microlitres of water. The crystalline monohydrate product was collected by filtration and air dried (yield 0.848 mg; 84%). Thermogravimetric analysis showed a loss of 1.21% weight to 80°C followed by a further loss of 1.76% to 125°C consistent with a monohydrate product.

## Claims

1. An intranasal pharmaceutical formulation for the treatment of male erectile dysfunction or female sexual disorders which comprises sildenafil mesylate, together with a pharmaceutically acceptable diluent or carrier in a form adapted for intranasal administration.

2. An intranasal formulation as claimed in claim 1 in the form of an aqueous solution or powder formulation.

3. An intranasal formulation as claimed in claim 1 comprising sildenafil mesylate in 5% weight/volume aqueous glycerine.

4. An intranasal formulation as claimed in claim 1 comprising sildenafil mesylate and caffeine in a buffered aqueous solution.

5. An intranasal formulation as claimed in claim 4 comprising sildenafil mesylate 100mg/ml and caffeine 15mg/ml, in an aqueous solution buffered to pH4.2.

6. An intranasal powder formulation as claimed in claim 1 comprising sildenafil mesylate having a particle size of from 5 to 40 micrometres optionally with a pharmaceutically acceptable carrier.

7. An intranasal pharmaceutical presentation comprising sildenafil mesylate together with a pharmaceutically acceptable diluent or carrier as claimed in any one of claims 1 to 6 in an intranasal delivery system or device.

8. The use of sildenafil mesylate for the manufacture of an intranasal medicament for the treatment of male erectile dysfunction or female sexual disorders.

9. Sildenafil mesylate.

10. Sildenafil mesylate, crystalline mono or dihydrate.

## Patentansprüche

1. Intranasale pharmazeutische Formulierung zur Behandlung von männlicher erektiler Dysfunktion oder weiblichen Sexualstörungen, die Sildenafilmesylat zusammen mit einem pharmazeutisch akzeptablen Verdünnungsmittel oder Träger in einer zur intranasalen Verabreichung angepassten Form umfasst.

2. Intranasale Formulierung gemäß Anspruch 1 in Form einer wässrigen Lösung oder einer Pulverformulierung.

3. Intranasale Formulierung gemäß Anspruch 1, die Sildenafilmesylat in 5%-igem (Gewicht/Volumen) wässrigem Glycerin umfasst.

4. Intranasale Formulierung gemäß Anspruch 1, die Sildenafilmesylat und Koffein in einer gepufferten wässrigen Lösung umfasst.

5. Intranasale Formulierung gemäß Anspruch 4, die 100 mg/ml Sildenafilmesylat und 15 mg/ml Koffein in einer auf einen pH-Wert von 4,2 gepufferten wässrigen Lösung umfasst.

6. Intranasale Pulverformulierung gemäß Anspruch 1, die Sildenafilmesylat mit einer Teilchengröße von 5 bis 40 µm optional mit einem pharmazeutisch akzeptablen Träger umfasst.

7. Intranasale pharmazeutische Zubereitung, die Sildenafilmesylat zusammen mit einem pharmazeutisch akzeptablen Verdünnungsmittel oder Träger gemäß einem der Ansprüche 1 bis 6 in einem intranasalen Abgabesystem oder einer intranasalen Abgabevorrichtung umfasst.

8. Verwendung von Sildenafilmesylat zur Herstellung eines intranasalen Medikaments zur Behandlung von männlicher erektiler Dysfunktion oder weiblichen Sexualstorungen.

9. Sildenafilmesylat.

10. Kristallines Mono- oder Dihydrat von Sildenafilmesylat.

## Revendications

1. Formulation pharmaceutique intranasale pour le traitement des dysfonctionnements de l'érection masculine ou de troubles sexuels féminins, qui comprend du mésylate de sildénafil, conjointement avec un diluant ou support pharmaceutiquement acceptable, sous une forme adaptée à l'administration intranasale.

2. Formulation intranasale suivant la revendication 1, sous forme d'une solution aqueuse ou d'une formulation en poudre.

3. Formulation intranasale suivant la revendication 1, comprenant du mésylate de sildénafil dans une solution aqueuse à 5% en poids/volume de glycérol.

4. Formulation intranasale suivant la revendication 1, comprenant du mésylate de sildénafil et de la caféine dans une solution aqueuse tamponnée.

5. Formulation intranasale suivant la revendication 4, comprenant du mésylate de sildénafil à 100 mg/ml et de la caféine à 15 mg/ml, dans une solution aqueuse tamponnée à pH 4,2.

6. Formulation de poudre intranasale suivant la revendication 1, comprenant du mésylate de sildénafil ayant un diamètre de particule de 5 à 40 micromètres, facultativement avec un support pharmaceutiquement acceptable.

7. Présentation pharmaceutique intranasale comprenant du mésylate de sildénafil conjointement avec un diluant ou support pharmaceutiquement acceptable, suivant l'une quelconque des revendications 1 à 6, dans un système ou dispositif d'administration intranasale.

8. Utilisation de mésylate de sildénafil pour la production d'un médicament intranasal destiné au traitement des dysfonctionnements de l'érection masculine ou de troubles sexuels féminins.

9. Mésylate de sildénafil.

10. Mono- ou dihydrate de mésylate de sildénafil cristallin.
